# EUROPÄISCHE PATENTANMELDUNG

(11) **EP 0 727 482 A2**
(43) Veröffentlichungstag der Anmeldung: **21.08.1996**
(21) Anmeldenummer: 95118260.9
(22) Anmeldetag: 21.11.1995
(51) Int. Cl.: C12N 5/02, C12N 5/06

(54) **Verfahren für die Kultivierung von Zellen auf einem Träger, Vorrichtung zur Durchführung des Verfahrens und Verwendung der Vorrichtung**

(30) Priorität: 15.02.1995 DE 19504958
(71) Anmelder: Heraeus Instruments GmbH, D-63450 Hanau (DE)
(72) Erfinder: Nagels, Hans-Otto, Dr., D-37120 Bovenden (DE); Schröder, Dieter, D-37520 Osterode (DE); Kopowski, Eckar, Dr., D-38102 Braunschweig (DE)
(74) Vertreter: Kühn, Hans-Christian

(57) **Zusammenfassung**

Es sind Verfahren und Vorrichtungen für die Kultivierung von Zellen auf einem Träger bekannt, bei denen ein dreidimensionaler Trägers für die zu kultivierende Zellkultur in einem Kulturgefäß, bereitgestellt und die Zellkultur mit Nährstoffen und mit Sauerstoff durch Umspülen mit einer Nähstoffe und Sauerstoff enthaltenden Nährstofflösung versorgt wird. Um hiervon ausgehend ein Verfahren anzugeben, mittels dem hohe Zelldichten ohne Gefahr von Kontaminationen erzielbar sind, eine dafür geeignete Vorrichtung zur Verfügung zu stellen, wird vorgeschlagen, daß der Träger in einer geschlossenen Zellkulturkammer bereitgestellt wird, wobei die Versorgung mit Sauerstoff durch eine die Zellkulturkammer begrenzende gasdurchlässige Gasaustauschmembran und die Versorgung mit Nährstoffen durch eine die Zellkulturkammer begrenzende Dialysemembran, durch die Nährstoffe transportiert und Stoffwechselprodukte abtransportiert werden, erfolgt.

## Beschreibung

Die Erfindung betrifft ein Verfahren für die Kultivierung von Zellen auf einem Träger durch Bereitstellen eines dreidimensionalen Trägers für die zu kultivierende Zellkultur in einem Kulturgefäß und Versorgen der Zellkultur mit Nährstoffen und mit Sauerstoff durch Umspülen des Trägers mit einer Nährstoffe und Sauerstoff enthaltenden Nährstofflösung. Weiterhin betrifft die Erfindung eine Vorrichtung für die Zellkultivierung, mit einem Kulturgefäß und mit einem darin angeordneten dreidimensionalen Träger für die zu kultivierende Zellkultur und eine Verwendung der Vorrichtung.

Derartige Verfahren werden für die Kultivierung sogenannter adhärenter Zellen eingesetzt. Diese Zellen vermehren sich nur auf einer festen Unterlage. Bei der Kultivierung solcher Zellen ist es daher erforderlich, einen geeigneten Träger zur Verfügung zu stellen. Auf diesem wachsen die Zellen üblicherweise in einer Mono-Lage. Es hat sich gezeigt, daß einige Zellarten für ein optimales Wachstum einen dreidimensionalen Trägerkörper benötigen. In der EP-B1 286 688 wird ein Verfahren für die Kultivierung derartiger Zellen und ein dafür geeigneter Träger beschrieben. Es wird ein Träger in Form eines aus ultrafeinen Fasern hergestellten Textilerzeugnisses vorgeschlagen. Das Textilerzeugnis ist aus den Fasern beispielsweise gestrickt, gewirkt oder gewebt. Für die Zellkultivierung wird der bekannte Träger sterilisiert und anschließend zusammen mit einer Nährmittellösung in eine Kunststoff-Petrischale gegeben. Diese Anordnung wird dann in einem Inkubator in einer geeigneten Atmosphäre behandelt.

Zwar gewährleistet die Porosität des bekannten Trägers ein optimales Zellwachstum sowie eine gute Versorgung der Zellen mit Nährstoffen, insbesondere mit Sauerstoff, und die Entsorgung von Schadstoffen. Es hat sich aber gezeigt, daß die erzielbare Zelldichte für viele Anwendungen nicht ausreichend ist. Zur Erzielung hoher Zelldichten auf dem Träger ist es erforderlich, das verbrauchte Nährmedium von Zeit zu Zeit auszutauschen. Hierbei müssen die zu kultivierenden Zellen aufwendig von dem Nährmedium abgetrennt werden. Besonders problematisch bei dem bekannten Verfahren und bei der bekannten Anordnung ist die Gefahr einer Kontamination der Zellen beim Kontakt mit den benötigten Arbeitsgerätschaften und während der Kultivierung im Inkubator.

Der Erfindung liegt daher die Aufgabe zugrunde, ein Verfahren anzugeben, mittels dem hohe Zelldichten ohne Gefahr von Kontaminationen erzielbar sind, eine dafür geeignete Vorrichtung zur Verfügung zu stellen sowie eine geeignete Verwendung anzugeben.

Hinsichtlich des Verfahrens wird diese Aufgabe ausgehend von dem eingangs genannten Verfahren erfindungsgemäß dadurch gelöst, daß der Träger in einer geschlossenen Zellkulturkammer bereitgestellt wird, wobei die Versorgung mit Sauerstoff durch eine die Zellkulturkammer begrenzende gasdurchlässige Gasaustauschmembran und die Versorgung mit Nährstoffen durch eine die Zellkulturkammer begrenzende Dialysemembran, durch die Nährstoffe transportiert und Stoffwechselprodukte abtransportiert werden, erfolgt.

Abgesehen von ihrer von der Dialysemembran gebildeten Begrenzung ist die Zellkulturkammer allseitig flüssigkeitsdicht verschlossen. Dadurch ist es möglich, die Zellkultur innerhalb eines flüssigen Versorgungsmedium intensiv zu bewegen, beispielsweise zu schaukeln oder zu rollen. Die intensive Bewegung erlaubt sowohl eine gute Versorgung der Zellkultur mit Nährstoffen und mit Sauerstoff als auch einen schnellen Abtransport von Schadstoffen.

Gleichzeitig verhindert die geschlossene Ausbildung der Zellkulturkammer das Einschleppen von Zellgiften. Durch die semipermeable Dialysemembran hindurch werden die Zellen von der Versorgungskammer aus kontinuierlich mit Nährstoffen versorgt, während Abbau- und Stoffwechselprodukte ebenfalls durch die Dialysemembran aus in die Versorgungskammer kontinuierlich abgeführt werden. Die Dialysemembran verhindert eine unkontrollierte Verteilung der Zellen innerhalb des Versorgungsmediums; sie begrenzt die Zellkultur innerhalb der Zellkulturkammer. Beim Austausch des Versorgungsmediums ist es deshalb nicht erforderlich, die Zellkultur vom Medium zu trennen. Die Gasversorgung der Zellen erfolgt ebenfalls kontinuierlich über die Dialysemembran und zusätzlich über die Gasaustauschmembran.

Es ist daher weder erforderlich, die Zellkulturkammer während der Kultivierung zum Einbringen neuer Nährstoffe zu öffnen, noch müssen in die Zellkulturkammer reichende Bauteile, wie Schläuche, Rohre oder Drehdurchführungen für die Gasversorgung der Zellen vorgesehen sein.

Da der Zellkulturkammer die für die Gasversorgung notwendigen Gase unmittelbar über die Gasaustauschmembran aus der Umgebungs-Atmosphäre zugeführt, bzw. die gasförmigen Stoffwechselprodukte durch die Gasaustauschmembran von der Zellkulturkammer direkt abgeführt werden, wird ein schneller und durch entsprechende Einstellung der die Zellkulturkammer umgebenden Atmosphäre und des Drucks außerhalb der Zellkulturkammer unmittelbar beeinflußbarer Gasaustausch erreicht.

Als Träger ist jeder dreidimensionale Körper geeignet, auf dem Zellen sich vermehren können. Es können mehrere einzelne Trägerkörper, beispielsweise Plättchen, Granulat oder Kügelchen vorgesehen sein. Der Träger kann auch aus einem dreidimensionalen, zusammenhängenden Netzwerk bestehen. Wichtig ist, daß die Geometrie des Trägers eine ausreichende Versorgung der daran anhaftenden Zellen mit Nährstoffen und mit Sauerstoff gewährleistet.

Als besonders günstig hat sich ein Verfahren erwiesen, bei dem das Kulturgefäß während der Kultivierung rotiert wird. Durch die Rotatiton wird eine gleichmäßige Versorgung der Zellkultur auf dem Träger mit Nährstoffen und mit Sauerstoff gewährleistet.

Es wird ein Verfahren bevorzugt, bei dem das Nährmedium in einer an die Zellkulturkammer angrenzenden Versorgungskammer bereitgestellt wird. Vorteilhafterwesie ist die Versorgungskammer, abgesehen von ihren Begrenzungsflächen, die von der Dialysemembran gebildet werden, flüssigkeitsdicht ausgebildet. Dadurch wird eine Kontamination des Versorgungsmediums während der Kultivierung verhindert. Die Zellkulturkammer und die Versorgungskammer können fest miteinander verbunden sein.

Besonders bewährt hat es sich, als Träger ein dreidimensionales Netzwerk aus Fasern oder aus Faserbruchstücken einzusetzen. Ein derartiges Netzwerk zeichnet sich durch eine große Oberfläche pro Volumeneinheit aus und erlaubt die Kultivierung hoher Zelldichten. Dabei kann die Oberfläche des Netzwerkes so strukturiert oder chemisch modifiziert sein, daß das Anhaften der zu kultivierenden Zellen gefördert wird.

Insbsondere im Hinblick auf einen Einsatz der Zellkultur mitsamt dem Träger im menschlichen oder tierischen Körper ist der Einsatz von Trägermaterial, das einen im menschlichen oer tierischen Körper abbaubaren Werkstoff enthält besonders geeignet.

Hinsichtlich der Vorrichtung wird die Aufgabe ausgehend von der eingangs genannten Vorrichtung erfindungsgemäß dadurch gelöst, daß der Träger in einer verschließbaren, teilweise von einer gasdurchlässigen Gasaustauschmembran begrenzten Zellkulturkammer angeordnet ist, an die eine ein Nährmedium aufnehmende Versorgungskammer angrenzt, wobei zwischen der Zellkulturkammer und der Versorgungskammer eine Dialysemembran angeordnet ist, durch die Nährstoffe von der Versorgungskammer in die Zellkulturkammer transportiert und Stoffwechselprodukte von der Zellkulturkammer in die Versorgungskammer abtransportiert werden.

Abgesehen von ihrer von der Dialysemembran gebildeten Begrenzung kann die Zellkulturkammer allseitig flüssigkeitsdicht verschließbar ausgebildet sein. Dadurch ist es möglich, die Zellkultur innerhalb eines flüssigen Versorgungsmedium intensiv zu bewegen, beispielsweise zu schaukeln oder zu rollen. Die intensive Bewegung gewährleistet sowohl eine gute Versorgung der Zellkultur mit Nährstoffen und mit Sauerstoff als auch einen schnellen Abtransport von Schadstoffen.

Gleichzeitig verhindert die geschlossene Ausbildung der Zellkulturkammer, das Einschleppen von Zellgiften. Durch die semipermeable Dialysemembran hindurch werden die Zellen von der Versorgungskammer aus kontinuierlich mit Nährstoffen versorgt, während Abbau- und Stoffwechselprodukte ebenfalls durch die Dialysemembran aus in die Vesorgungskammer kontinuierlich abgeführt werden. Die Gasversorgung der Zellen erfolgt ebenfalls kontinuierlich über die Dialysemembran und zusätzlich über die Gasaustauschmembran. Es ist daher weder erforderlich, die Zellkulturkammer während der Kultivierung zum Einbringen neuer Nährstoffe zu öffnen, noch müssen in die Zellkulturkammer hineinreichende Bauteile, wie Schläuche, Rohre oder Drehdurchführungen für die Gasversorgung der Zellen vorgesehen sein. Deartige Bauteile bilden eine zusätzliche Kontaminationsquelle.

Die Gesamtfläche, das Material und die Dicke der Gasaustauschmembran sind so zu wählen, daß eine Sauerstoffversorgung gewährleistet wird, die den Sauerstoffbedarf bei den angestrebten hohen Zelldichten deckt. Geeignete Gasaustauschmembran-Geometrien und Gasdurchlässigkeiten lassen sich anhand weniger Versuche ermitteln. Geeignete Materialien weisen für Sauerstoff einen Durchlässigkeitskoeffizienten von mindestens 1 x 10¹⁹ m²/s x Pa, vorteilhafterweise mindestens 5 x 10¹⁹ m²/s x Pa, auf. Als besonders gut geeignete Materialien zur Ausbildung der Gasaustauschmembran haben sich Silikongummi und mikroporöses, hydrophobes oder hydrophobiertes Material, wie Polytretrafluorethylen, erwiesen. Um eine ausreichende Sauerstoffversorgung zu gewährleisten, werden möglichst dünne Gasaustauschmembranen bevorzugt. Bewährt haben sich Membranen mit einer Dicke zwischen 0,1 mm und 1 mm.

Als Träger ist jeder dreidimensionale Körper geeignet, auf dem Zellen sich vermehren können. Es können mehrere einzelne Trägerkörper, beispielsweise Kügelchen, Granulat oder Plättchen vorgesehen sein. Durch Bewegen der Zellkulturkammer können derartige Träger in Suspension gehalten werden. Eine Kultivierung in einer Suspension ermöglicht eine besonders gute Versorgung der an den Trägerkörpern anhaftenden Zellen mit Sauerstoff und mit Nährstoffen. Das spezifische Gewicht der Trägerkörper kann so gewählt sein, daß sie in der Nährmittellösung schwimmen. Dies erleichtert die Stabilisierung der Suspension. Der Träger kann auch aus einem dreidimensionalen, zusammenhängenden Netzwerk bestehen. Wichtig ist, daß die Geometrie des Trägers eine ausreichende Versorgung der daran anhaftenden Zellen mit Nährstoffen und mit Sauerstoff durch Umspülen mit nährstoff- und sauerstoffhaltiger Nährmittellösung kontaminationsfrei erlaubt.

Als besonders vorteilhaft hat sich eine Vorrichtung erwiesen, die nach Art einer Rollflasche um eine Rotationsachse rollbar ausgebildet ist. Vorteilhafterweise sind die Zellkulturkammer und die Versorgungskammer fest aber lösbar miteinander verbunden. Das Nährmedium wird dabei in einer an die Zellkulturkammer angrenzenden Versorgungskammer bereitgestellt, die abgesehen von ihren Begrenzungsflächen, die von der Dialysemembran gebildet werden, flüssigkeitsdicht ausgebildet ist. Dadurch wird eine Kontamination des Versorgungsmediums während der Kultivierung verhindert. Notwendige Öffnungen, beispielsweise in Form von Septen, zur Entnahme von Nährmedium oder von Proben der Zellkultur sind daher verschließbar ausgebildet. Durch die Rollbewegung wird das Nährmedium innerhalb und außerhalb der Zellkulturkammer durchmischt und damit eine besonders gute Versorgung der Zellkultur mit Sauerstoff und mit Nähstoffen erreicht. Zur Unterstützung der Durchmischung können in der Versorgungskammer Mischelemente, beispielsweise in Form von an den Begrenzungswänden der Versorgungskammer angeordneten Flächenteilen oder in Form von Mischkugeln vorgesehen sein. Auch eine Lufblase trägt zur Durchmischung des Nährmediums bei. Eine Luftblase kann sowohl in der Versorgungskammer als auch in der Zellkulturkammer vorhanden sein.

Besonders bewährt hat es sich, als Träger ein dreidimensionales Netzwerk aus Fasern oder aus Faserbruchstücken vorzusehen. Die Fasern können massiv oder auch hohl sein. Bei hohlen Fasern steht den Zellen eine zusätzliche Oberfläche zur Verfügung. Das Netzwerk kann in die Zellkulturkammer eingelegt sein, beispielsweise in Form einer Matte, die auf die passende Größe zurechtgeschnitten wird. Es kann auch direkt in der Zellkulturkammer erzeugt werden, beispielsweise durch Verspinnen von Fasern in die Zellkulturkammer. Besonders bewährt hat sich ein Netzwerk, mit einer mechanischen Stabilität, die es erlaubt, daß es bei der Herstellung der Zellkulturkammer aus einem thermoplastischen Kunststoff durch Spritzguß mit verspritzt werden kann. Als besonders günstig hat sich auch ein Träger erwiesen, der aus einem Filtermaterial oder aus einem Membran-Material besteht. Es ist günstig, die Oberfläche des Netzwerkes so zu modifizieren, daß die Zellen besonders leicht anhaften und sich schneller vermehren. Dies kann durch eine geeignete Oberflächenstruktur als auch durch eine Beschichtung, beispielsweise mit Collagen, bewirkt werden.

Als besonders vorteilhaft hat sich ein Träger erwiesen, der aus mehreren, voneinander lösbaren Schichten aufgebaut ist. Ein derartiger Träger ermöglicht nach Abschluß der Zellkultivierung die Entnahme einzelner Schichten, die jeweils mit der Zellkultur belegt sind. Diese Schichten können mit Hautzellen belegt sein und beispielsweise als Hautersatz bei Transpantationen eingesetzt werden. Der besondere Vorteil eines derartigen Trägers besteht auch darin, daß bei einer einzigen Zellkultur eine große Hautersatz-Fläche gleichzeitig erzeugt werden kann. Dabei hat sich ein Träger besonders bewährt, der aus einem biologisch abbaubaren Material besteht.

Besonders bewährt hat sich eine Ausführungsform der Vorrichtung, bei der der Träger in Form eines an die Gasaustauschmemebran angrenzenden, dreidimensionalen, porösen Netzwerkes ausgebildet ist. Bei dieser Ausführungsform werden die auf dem Träger anhaftenden Zellen besonders gut mit Sauerstoff versorgt. Das Netzwerk kann mattenförmig ausgebildet sein. Die Matte kann aus einem Stück bestehen oder aus mehreren Einzelteilen, beispielsweise aus Sechseckteilen zusammengesetzt sein.

Insbesondere Im Hinblick auf die Herstellung von Hauttransplantaten hat sich ein Träger in Form eines aus mindestens zwei porösen Schichten gebildeten Bauteils bewährt, wobei jeweils die Schichten paarweise gegenüberliegen. Die Schichten sind voneiander lösbar und liegen mit ihren Planseiten aufeinander. Die Zellen haften in erster Linie an der freien Oberfläche an. Durch schrittweisen Aufbau mit verschiedenen Zelltypen kann auf dieser Oberfläche dann beispielsweise der Zell-Aufbau der Haut künstlich nachgebildet werden, während die gegenüberliegende Seite der Schicht frei von Zellen bleibt. Um eine Bevölkerung der sich gegenüberliegenden Planseiten mit Zellen vollständig zu vermeiden, ist eine Ausführungsform der Doppelschicht vorteilhaft, bei der die Schichten dicht aufeinanderliegen oder einen Hohlraum einschließen, wobei die Porosität der Schichten derart eingestellt ist, daß die zu kultivierenden Zellen nicht zwischen die Schichten bzw. nicht in den Hohlraum gelangen. Alternativ oder zusätzlich können die sich gegenüberliegenden Oberflächen der Schichten hydrophob ausgebildet sein. Hierzu kann eine Hydrophobierung durch eine Beschichtung, beispielsweise mit Polypropylen, erfolgen, oder es kann ein die Schicht überdeckendes Trennvlies vorgesehen sein. Dadurch wird das Zellwachstum auf diesen Schichten gehemmt.

Als vorteilhaft hat sich eine Ausführungsform der erfindungsgemäßen Vorrichtung erwiesen, bei der die Gasaustauschmembran von der Zellkulturkammer lösbar ausgebildet ist. Dadurch ist es möglich, nach Beendigung der Zellkultivierung die Zellkulturkammer durch Entfernen der Gasaustauschmembran zu öffnen und die mit den Zellen bewachsenen Träger zu entfernen.

Als günstig hat es sich erwiesen, die Gasaustauschmembran mit einer Abzieheinrichtung zu versehen. Hiermit ist die Gasaustauschmembran durch Abziehen besonders einfach zu entfernen. Die Abzieheinrichtung umfaß vorteilhafterweise eine am Rand der Gasaustauschmembran umlaufende Soll-Abreißkante. Die Ausbildung einer derartigen Soll-Abreißkante bei Kunststoffen ist dem Fachmann geläufig. Sie kann beispielsweise dadurch realisiert werden, daß die Gasaustauschmembran an ihrem Rand mit einer umlaufenden Verdickung ausgebildet ist, die in Richtung auf die Abreißkante spitz zuläuft und die mit einer ebenso ausgebildeten Verdickung auf der gegenüberliegenden Seite der Abreißkante korrespondiert.

Besonders bewährt hat sich eine Ausführungsform der Vorrichtung, bei der der Träger auf einem Stützteil gehalten wird. Dadurch kann der Träger selbst sehr dünnwandig ausgebildet sein. Die mechanische Stabilität übernimmt wird durch das Stützteil erreicht. Vorteilhafterweise ist das Stützteil als flächiges Stützgitter ausgebildet, auf dem Befestigungsstellen für den Träger vorgesehen sind. Durch die Öffnungen ii der Gitterstruktur des Stützteils wird eine ausreichende Versorgung der auf dem Träger wachsenden Zellen gewährleistet. Für flächig ausgebildete Träger, die -abgesehen von mit den Bestestigungsstellen korrespondierenden Halteflächen-, in Form eines regelmäßigen Sechseckes ausgebildet sind, hat sich ein Stützteil bewährt, bei dem die Befestigungsstellen außerhalb der Sechseckfläche des Trägers angeordnet sind. Die Halteflächen des Trägers befinden sich ebenfalls außerhalb der Sechseckfläche und sie korrespondieren mit den Befestigungsstellen. Die Befestigung des Trägers auf dem Stützteil erfolgt beispielsweise durch Anschweißen. An den Schweißstellen wird der Träger jedoch üblicherweise unbrauchbar für die Zellkultivierung. Daher werden die Halteflächen möglicht klein gewählt. Dadurch, daß die Träger in Form eines regelmäßigen Sechseckes ausgebildet sind, wird durch Aneinanderreihung mehrerer Träger eine lückenlose Ausfüllung einer größeren Fläche ermöglicht. Die Halteflächen, auf denen der Träger auf dem Stützteil befestigt ist, liegen außerhalb der Sechseckfläche und werden abgeschnitten. Selbstverständlich ist eine lückenlose größere Fläche auch durch Aneinanderreihung von Trägern mit dreieckiger oder rechteckiger Form herstellbar. Die Sechseckform hat den weiteren Vorteil, daß sie einen runden Querschnitt, wie beispielsweise bei einer Rollerflasche, besser ausfüllt.

Es hat sich gezeigt, daß eine erfindungsgemäße Vorrichtung, insbesondere mit einem Träger in Form eines dreidimensionalen, porösen Netzwerkes, nach einer Kultivierung von Hautzellen gemäß dem erfindungsgemäßen Verfahren für die Herstellung von Hauttransplantaten besonders gut geeignet ist.

Ausführungsbeispiele der Erfindung sind in der Zeichnung dargestellt und werden nachstehend näher erläutert. In der Zeichnung zeigen im einzelnen:
- **Figur 1**: eine auf einem Ständer angeordnete, erfindungsgemäße Vorrichtung in einer dreidimensionalen, schematischen Darstellung,
- **Figur 2**: einen Schnitt durch eine Zellkulturkammer mit einem darin angeordneten Träger,
- **Figur 3**: eine schematische Darstellung einer Draufsicht auf eine Zellkulturkammer mit einer entfernbaren Gasaustauschmembran,
- **Figur 4**: die Zellkulturkammer gemäß Figur 3 mit teilweise abgezogener Gasaustauschmembran und
- **Figur 5**: in schematischer Darstellung ein Stützgitter für die Aufnahme eines Trägers.

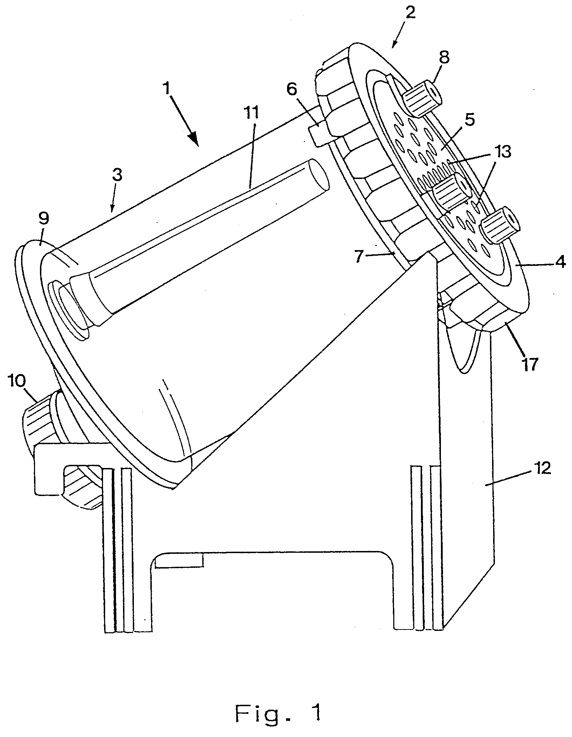

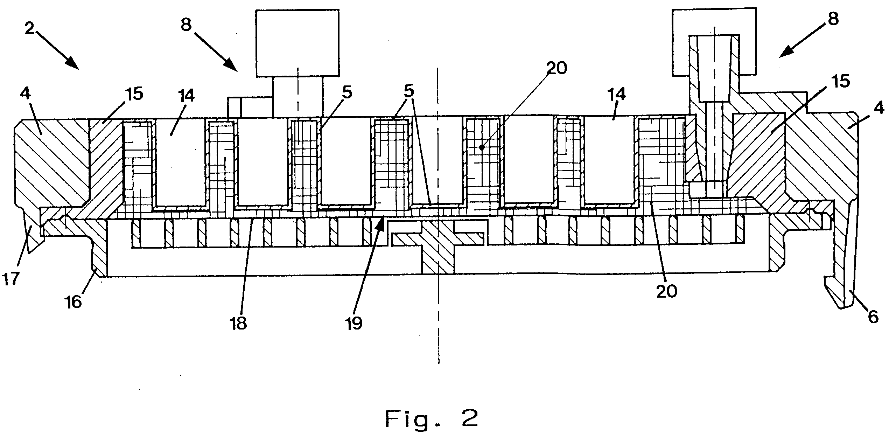

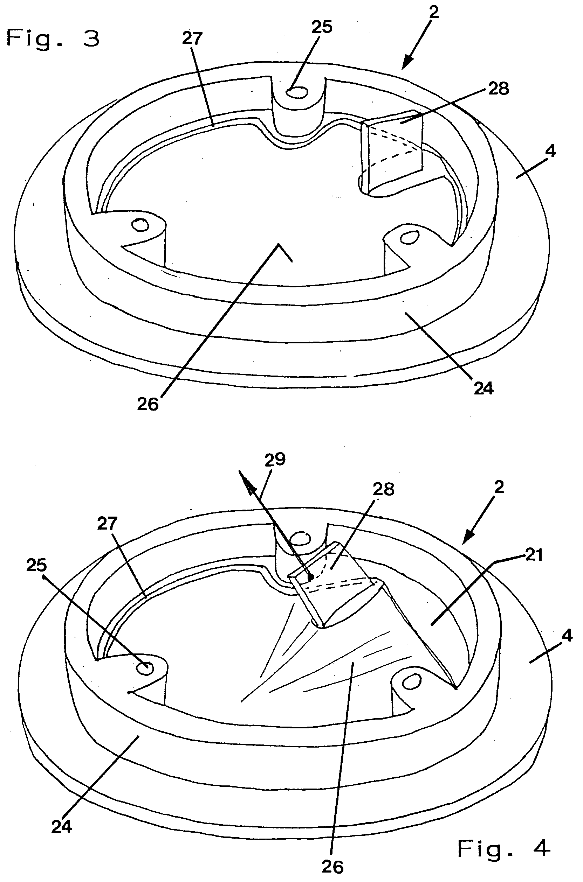

Bei der in **Figur 1** dargestellten Ausführungsform der erfindungsgemäßen Vorrichtung ist die Bezugsziffer 1 einem rollflaschenähnlichen Kulturgefäß 1 insgesamt zugeordnet. Das im wesentlichen zylinderförmige Kulturgefäß 1 ist modulartig aus einem Produktionsmodul 2 und aus einem Versorgungsmodul 3 aufgebaut. Das Produktionsmodul 2 ist in Form eines ca. 1 cm hohen Kreisringes 4 aus einem stabilen Kunststoff gebildet, dessen nach außen weisende Stirnseite von einer Silikonmembran 5 und dessen dem Versorgungsmodul 3 zugewandten Stirnseite von einer (in den Figuren 2 und 3 dargestellten) Dialysemembran 18 überspannt werden. Zur Halterung des Produktionsmoduls 2 an dem Versorgungsmodul 3 ist der Kreisring 4 mit Schnapphaken 6 versehen, die über einem umlaufenden Ringwulst 7 des oberen Randes des Versorgungsmoduls 3 einrasten. Im eingerasteten Zustand stoßen das Produktionsmodul 2 und das Versorgungsmodul 3 mit ihren Stirnseiten aneinander. Dabei gewährleistet ein zwischen den Stirnseiten angeordneter (nicht dargestellter) Dichtring eine gas- und flüssigkeitsdichte Verbindung von Produktionsmodul 2 und Versorgungsmodul 3. Das Befüllen des Produktionsmoduls 2 mit einer Zellsuspension geschieht durch einen der drei Luer-Lock-Anschlüsse 8, die mittels Kappen 9 gas- und flüssigkeitsdicht verschließbar sind. Der untere Rand des Versorgungsmoduls 3 ist mit einer über die Zylinderfläche vorstehenden, umlaufenden Abrollkante 10 versehen. Gleichermaßen wirkt der Kreisring 4 des Produktionsmoduls 2, der mit einem glatten umlaufenden und ebenfalls über der Zylinderfläche des Versorgungsmoduls 3 vorstehenden Rand ausgebildet ist. Beim Rollen um seine Längsachse liegt das Kulturgefäß 1 nur auf der Abrollkante 10 und dem Kreisring 4 auf; die Zylinderfläche des Versorgungsmoduls wird dabei nicht beansprucht. Zum Befüllen des Versorgungsmoduls 3 mit einer Nährstofflösung für die zu kultivierenden Zellen ist dieses mit einer durch eine Schraubkappe 10 verschließbaren Einfüllöffnung versehen.

Das Versorgungsmodul 3 ist mit einem schlauchartigen Druckfinger 12 aus Silikon ausgestattet. Dieser ragt ausgehend von der dem Produktionsmodul 2 abgewandten Stirnseite des Versorgungsmoduls 3 annähernd parallel zu dessen Längsachse in das Innere des Versorgungsmoduls 3 hinein. Dabei verläuft die Längsachse des Druckfingers 12 nicht in der Längsachse des Versorgungsmoduls, die auch die Rotationsachse für das Kulturgefäß 1 darstellt, sondern daneben. Das Kulturgefäß ist auf einem Ständer 13 abgelegt. Die Silikonmembran 5 hat eine Dicke von ca. 0,2 mm. Sie ist mit im Querschnitt kreisrunden, in das Produktionsmodul 2 hineinragenden und nach außen offenen Noppen 14 versehen, wie sie aus der Schnittdarstellungen der Figuren 2 deutlicher ersichtlich sind. Der Randbereich 15 der Silikonmembran 5 ist verdickt ausgebildet. Die Silikonmembran 5 ist im Randbereich 15 zwischen einem Stützgitter 16 und dem Kreisring 4 eingeklemmt. Hierzu ist der Kreisring 4 mit an seinem Umfang gleichmäßig verteilten Haken 17 versehen, die über den Rand des Stützgitters 16 greifen. Das Stützgitter 16 dient als mechanischer Schutz und als Stütze für die dünne Dialysemembran 18, die mit ihrem Rand auf dem Randbereich 15 der Silikonmembran 5 aufliegt und die ebenfalls zwischen dem Stützgitter 16 und dem Kreisring 4 eingeklemmt ist.

Bei der in **Figur 2** ausschnittsweise dargestellten Ausführungsform der erfindungsgemäßen Vorrichtung ist für die zu kultivierende Zellkultur ein Träger in Form einer Matte 19 vorgesehen. Die Matte 19 ist aus einem dreidimensionalen Netzwerkes aus miteinander verfilzten Fasern 20 gebildet und füllt die Zellkulturkammer 21 innerhalb des Produktionsmoduls 2 vollständig aus. In alternativen Ausführungsformen können die Fasern 20 auch miteinander verwirkt, verstrickt oder verwebt sein. Wichtig ist bei derartigen Ausführungsformen des Trägers, daß die Matte 19 eine mechanische Stabilität aufweist, die ein Ausstanzen oder Ausschneiden von Öffnungen, entsprechend die Zellkulturkammer 21 hineinragenden Noppen 14, erlaubt. Die Dicke der Matte entspricht etwa der Höhe der Zellkulturkammer 21, in die sie leicht entnehmbar eingelegt ist. Die Fasern 20 bestehen aus Polyhydrxybuttersäure, die im menschlichen Körper leicht abbaubar ist. Die Nährstoffe für die bei der Zellkutivierung auf der Matte 19 wachsenden Zellen werden im der Zellkulturkammer 21 benachbarten Versorgungsmodul (in der Figur 2 nicht dagestellt) bereitgestellt.

In einem weiteren Ausführungsbeispiel ist ein Träger in Form einer "Doppelschicht" aus zwei planparallel zueinander angeordneten Schichten vorgesehen. Die Schichten bestehen aus faseriger Polyhydroxibuttersäure, also aus einem biologisch abbaubaren Material. Die Porenweite der Schichten ist wesentlich kleiner als die mittlere Zellgröße humaner Fibroplasten eingestellt. An ihren Stirnseiten sind die Schichten unter Bildung eines Hohlraumes miteinander verschweißt. Die so thermisch vorbelasteten Schweißstellen lassen sich später durch Hydrolyse schnell auflösen und die Schichten sich so leicht voneinander trennen. Duch Zugabe humaner Fibroplasten bildet sich auf den dem Hohlraum abgewandten Oberflächen des Trägers eine entsprechende Monolayer, die als Basis für das Wachstum weiterer Zellen, wie menschlicher Keratinozyten, dient. Durch sukzessive Zugabe entsprechender Zellkulturen wird so auf dem Träger der Aufbau menschlicher Haut künstlich nachgebildet. Nach Abschluß der Kultivierung wird die Doppelschicht getrennt, indem die leicht hydrolisierbare Schweißstellen aufgelöst werden. Die einzelnen Schichten sind dann als Hauttransplantat einsetzbar.

Nachfolgend wird ein weiteres Beispiel für ein erfindungsgemäßes Verfahren zur Zellkultivierung mittels der beschriebenen Vorrichtung näher erläutert.

Die zu kultivierenden Zellen werden in einem flüssigen Medium in das Produktionsmodul 1 eingefüllt, das ein Volumen von ca. 35 ml aufweist. In das Produktionsmodul 1 ist ein Träger in Form einer biegsamen Matte 19 eingesetzt. Die Matte 19 ist aus einem dreidimensionalen Netzwerkes aus miteinander verfilzten Fasern 20 gebildet. Das wesentlich größere Versorgungsmodul 2 (ca. 600 ml) wird mit dem Nährmedium zu 2/3 gefüllt.

Durch die semipermeable Dialysemembran 18 können weder die Zellen noch die von den Zellen freigesetzten hochmolekularen Produkte passieren. Nährstoffe, Vitamine, Ionen und im Medium physikalisch gelöste Gase (O₂, CO₂) können dagegen aus dem Versorgungsmodul 3 nahezu ungehindert in das Produktionsmodul 2 gelangen und - wegen des etwa 10-fachen Überschusses an Versorgungsmedium - die Zellen über einen langen Zeitraum (in Abhängigkeit von der Zelldichte) mit den für die Kultivierung benötigten Stoffen versorgen. Gleichzeitig können von den Zellen abgegebene saure, toxische und andere Metabolite durch die Dialysemembran 18 das Produktionsmodul 2 verlassen und in dem wesentlich größeren Volumen des Versorgungsmoduls 3 aufgefangen und neutralisiert werden. Dadurch sind hohe Zelldichten und hohe Dichten an Zellprodukten erreichbar.

Um eine optimale Ver- und Entsorgung der Zellkultur zu erreichen, müssen die Zellen ständig von Nährmittellösung umspült sein. Dies wird durch eine Rotation des Kulturgefäßes 1 um seine Längsachse erreicht, wodurch außerdem ein optimaler Stoffaustausch an der Dialysemembran 18 und an der Silikonmembran 5 gewährleistet wird. Durch die netzwerkartige, poröse Struktur der Matte 19 wird jede Faser 20 von der Nährmittellösung erreicht.

Für einen optimalen Stoffaustausch an der Dialysemembran 18 und an der Silikonmembran 5 sorgt auch der in das Versorgungsmodul 3 hineinreichende mit einer dünnen Silikonmembran überzogene Druckfinger 11. Dieser fängt bei der Zellkultivierung entstehende Druckschwankungen auf und ermöglicht gleichzeitig den Gasaustausch zwischen Versorgungsmodul 3 und Brutschrankatmosphäre. Seine exzentrische Anordnung außerhalb der Rotationsachse dient auch der besseren Durchmischung des Nährmediums. Länge und Durchmesser des Druckfingers 11 sind an die zu erwartenden maximalen Druckschwankungen angepaßt. Bei Überdruck innerhalb des Versorgungsmoduls 3 wird der Druckfinger 11 zusammengepreßt. Dadurch wird ein Ausbeulen der Dialysemembran 18 und der Silikonmembran 5 und damit eine Änderung des Volumens des Produktionsmoduls 2 sowie der Mischbedingungen vermieden. Der Druckfinger 11 gewährleistet so eine reproduzierbare Zellkultur.

Die Fasern 20 der Matte 19 sind nach abgeschlossener Zellkutivierung mit den zu kultivierenden Zellen belegt. Die Matte 19 kann daraufhin dem Zellkulturgefäß 1 entnommen und beispielsweise als Hauttransplantat unmittelbar verwendet werden.

Die in den **Figuren 3 und 4** dargestellte Ausführungsform des Produktionsmoduls 2 ist besonders geeignet für die erfindungsgemäße Kultivierung unter Verwendung eines Trägers in Form einer aus mehreren Einzelschichten gebildeten Matte, wobei die Einzelschichten leicht voneinander lösbar sind nach der Kultivierung einzeln aus der Zellkulturkammer entnommen werden können. Die Einzelschichten der Matte sind aus einem Netzwerk von Fasern aus Polyhydrxybuttersäure gebildet. Die Fasern werden vor ihrem Einbau in die Zellkulturkammer mit menschlichen Fibroplasten beschichtet. Diese Beschichtung dient als Basis für das Anwachsen anderer Zellen.

Das Produktionsmodul 2 weist einen stabilen Kreisring 4 auf, der an seinem inneren Rand mit einem umlaufenden Wulst 24 versehen ist, in dem die Anschlußöffnungen 25 für die Luer-Lock-Anschlüsse (siehe Figur 2) ausgebildet sind. Zur Erleichterung der Entnahme der Einzelschichten aus der Zellkulturkammer (siehe Figur 4) ist die dem Gasaustausch dienende Silikonmembran 26 im Bereich des Wulstes 24 mit einer umlaufenden Abreißkante 27 und mit einer Abziehlasche 28 versehen.

Das Entfernen der Silikonmembran 26 ist in **Figur 4** dargestellt. Hierzu wird einfach an der Abziehlasche 28 entlang der mit dem Richtungspfeil 29 bezeichneten Richtung gezogen. Dadurch reißt die Silikonmembran 26 entlang der Abreißkante 27 auf und kann vollständig entfernt werden. Das Innere der Zellkulturkammer 21 ist dann frei zugänglich und die mit der Zellkultur bewachsenen Einzelschichten können daraus entnommen werden.

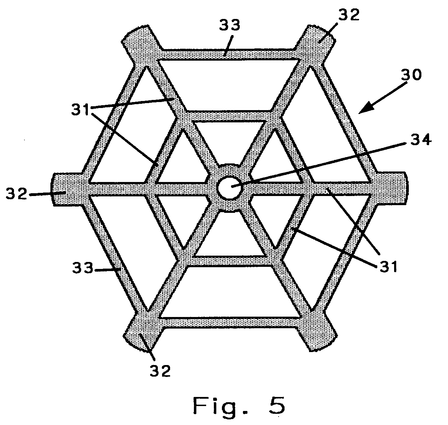

Das in der **Figur 5** dargestellte Stützgitter 30 ist besonders geeignet für die Kultivierung von Trägern in Form flexibler Membranen (in der Figur 5 nicht dargestellt), die keine ausreichende mechanische Stabilität aufweisen. Das Stützgitter 30 besteht aus miteinander verbundenen Stegen 31 aus einem schweißbaren Kunststoff, die ein regelmäßiges Sechseck umfassen. Für die Zellkultivierung werden die Membranen auf das Stützgitter 30 geschweißt. Hierzu sind auf dem Stützgitter 30 insgesamt sechs Schweißbereiche 32 vorgesehen, die um das Stützgitter 30 herum in einem regelmäßigen Sechseck angeordnet sind. Das Sechseck der Schweißbereiche 32 liegt dabei außerhalb der äußersten Sechseck-Stege 33 des Stützgitters 30. Die auf dem Stützgitter 30 zu haltende Membranen weist ebenfalls Sechseckstruktur mit einer dem Stützgitter 30 etwa entsprechenden Größe auf. Ebenso wie das Stützgitter 30 ist auch die Membran mit Schweißflächen versehen, die außerhalb seiner Sechseckfläche liegen und deren geometrische Anordnung und Größe den Schweißbereichen 32 entspricht. Innerhalb des Produktionsmoduls (nicht dargestellt) sind mehrere dieser Stützgitter 30 stapelweise angeordnet. Um ein koaxiales Stapeln zu erleichtern ist jedes Stützgitter 30 mit einer zentrischen Haltebohrung 34 versehen. Der Abstand benachbarter Stützgitter 30 liegt im Bereich eines Millimeters. Die Stapelung der Stützgitter im Produktionsmodul erlaubt eine Zellkultivierung auf dünnwandigen Membranen auf engstem Raum, ohne daß sich die Membranen berühren oder die Zellkulturen benachbarter Membranen miteinander verwachsen.

## Patentansprüche

1. Verfahren für die Kultivierung von Zellen auf einem Träger durch Bereitstellen eines dreidimensionalen Trägers für die zu kultivierende Zellkultur in einem Kulturgefäß, und Versorgen der Zellkultur mit Nährstoffen und mit Sauerstoff durch Umspülen mit einer Nähstoffe und Sauerstoff enthaltenden Nährstofflösung, dadurch gekennzeichnet, daß der Träger in einer geschlossenen Zellkulturkammer bereitgestellt wird, wobei die Versorgung mit Sauerstoff durch eine die Zellkulturkammer (2) begrenzende gasdurchlässige Gasaustauschmembran (5) und die Versorgung mit Nährstoffen durch eine die Zellkulturkammer (2) begrenzende Dialysemembran (18), durch die Nährstoffe transportiert und Stoffwechselprodukte abtransportiert werden, erfolgt.

2. Verfahren nach Anspruch 1, dadurch gekennzeichnet, daß das Kulturgefäß (1) während der Kultivierung rotiert wird.

3. Verfahren nach Anspruch 1 oder 2, dadurch gekennzeichnet, daß das Nährmedium in einer an die Zellkulturkammer (2) angrenzenden Versorgungskammer (3) bereitgestellt wird.

4. Verfahren nach einem der vorhergehenden Ansprüche, dadurch gekennzeichnet, daß als Träger (19) ein dreidimensionales Netzwerk aus Fasern oder aus Faserbruchstücken eingesetzt wird.

5. Vorrichtung für die Zellkultivierung, mit einem Kulturgefäß und mit einem darin angeordneten dreidimensionalen Träger für die zu kultivierende Zellkultur, dadurch gekennzeichnet, daß der Träger (19) in einer verschließbaren, teilweise von einer gasdurchlässigen Gasaustauschmembran (5) begrenzten Zellkulturkammer (2) angeordnet ist, an die eine ein Nährmedium aufnehmende Versorgungskammer (3) angrenzt, wobei zwischen der Zellkulturkammer (2) und der Versorgungskammer (3) eine Dialysemembran (18) angeordnet ist, durch die Nährstoffe von der Versorgungskammer (3) in die Zellkulturkammer (2) transportiert und Stoffwechselprodukte von der Zellkulturkammer (2)in die Versorgungskammer (3) abtransportiert werden.

6. Vorrichtung nach Anspruch 5, dadurch gekennzeichnet, daß sie um eine Rotationsachse rollbar ausgebildet ist.

7. Vorrichtung nach einem der Ansprüche 5 bis 6, dadurch gekennzeichnet, daß als Träger (19) ein dreidimensionales Netzwerk aus Fasern (20) oder aus Faserbruchstücken vorgesehen ist.

8. Vorrichtung nach einem der Ansprüche 5 bis 7, dadurch gekennzeichnet, daß als Träger (19) ein aus mindestens zwei porösen Schichten gebildetes Bauteil vorgesehen ist, wobei jeweils die Schichten paarweise gegenüberliegen.

9. Vorrichtung nach Anspruch 8, dadurch gekennzeichnet, daß die Schichten dicht aufeinanderliegen oder einen Hohlraum einschließen und die Porosität der Schichten derart eingestellt ist, daß die zu kultivierenden Zellen nicht zwischen die Schichten bzw. in den Hohlraum gelangen.

10. Vorrichtung nach Anspruch 8 oder 9, dadurch gekennzeichnet, daß die sich gegenüberliegenden Oberflächen der Schichten hydrophob ausgebildet sind.

11. Vorrichtung nach einem oder mehreren der Ansprüche 5 bis 10, dadurch gekennzeichnet, daß der Träger (19) lösbar in die Zellkulturkammer (2) eingesetzt ist.

12. Vorrichtung nach einem oder mehreren der Ansprüche 5 bis 11, dadurch gekennzeichnet, daß der Träger in Form einer stabilen, mit Kunststoff umspritzbaren Matte (19) ausgebildet ist.

13. Vorrichtung nach einem oder mehreren der Ansprüche 5 bis 12, dadurch gekennzeichnet, daß als Träger (19) ein Filtermaterial oder ein Membran-Material vorgesehen ist.

14. Vorrichtung nach einem oder mehreren der vorhergehenden Ansprüche 5 bis 13, dadurch gekennzeichnet, daß der Träger (19) eine das Zellwachstum fördernde Beschichtung aufweist und/oder die Oberfläche des Trägers (19) derart chemisch modifiziert ist.

15. Vorrichtung nach einem der Ansprüche 5 bis 14, dadurch gekennzeichnet, daß der Träger (19) aus mehreren, voneinander lösbaren Schichten aufgebaut ist.

16. Vorrichtung nach einem der Ansprüche 5 bis 15, dadurch gekennzeichnet, daß der Träger (19) aus einem im menschlichen Körper abbaubaren Werkstoff besteht.

17. Vorrichtung nach einem der Ansprüche 5 bis 16, dadurch gekennzeichnet, daß der Träger (19) in Form eines an die Gasaustauschmemebran (5) angrenzenden, dreidimensionalen, porösen Netzwerkes ausgebildet ist.

18. Vorrichtung nach einem der Ansprüche 5 bis 17, dadurch gekennzeichnet, daß die Gasaustauschmembran (5) von der Zellkulturkammer (21) lösbar ausgebildet ist.

19. Vorrichtung nach Anspruch 18, dadurch gekennzeichnet, daß die Gasaustauschmembran (5) mit einer Abzieheinrichtung (27; 28) versehen ist.

20. Vorrichtung nach Anspruch 19, dadurch gekennzeichnet, daß die Abzieheinrichtung eine am Rand der Gasaustauschmembran (5) umlaufende Soll-Abreißkante (27) umfaßt.

21. Vorrichtung nach einem der Ansprüche 5 bis 20, dadurch gekennzeichnet, daß der Träger (19) auf einem Stützteil (30) gehalten wird.

22. Vorrichtung nach Anspruch 21, dadurch gekennzeichnet, daß das Stützteil als flächiges Stützgitter (30) ausgebildet ist, auf dem Befestigungsstellen (32) für den Träger vorgesehen sind.

23. Vorrichtung nach Anspruch 22, dadurch gekennzeichnet, daß der Träger flächig und, abgesehen von mit den Bestestigungsstellen (32) korrespondierenden Halteflächen, in Form eines regelmäßigen Sechseckes ausgebildet ist, und daß die Halteflächen und die korrespondierenden Befestigungsstellen (32) außerhalb der Sechseckfläche des Trägers angeordnet sind.

24. Verwendung einer Vorrichtung nach einem der Ansprüche 5 bis 23 für die Herstellung von Hauttransplantaten durch Kultivierung von Hautzellen auf dem Träger (19) gemäß dem Verfahren nach einem der Ansprüche 1 bis 4.
